(19) Europäisches Patentamt

European Patent Office

Office européen des brevets

(11) **EP 1 039 394 A2**

(12) **EUROPEAN PATENT APPLICATION**

(43) Date of publication:
27.09.2000 Bulletin 2000/39

(51) Int. Cl.[7]: **G06F 17/00**

(21) Application number: **00105924.5**

(22) Date of filing: **23.03.2000**

(84) Designated Contracting States:
**AT BE CH CY DE DK ES FI FR GB GR IE IT LI LU MC NL PT SE**
Designated Extension States:
**AL LT LV MK RO SI**

(30) Priority: **23.03.1999 JP 7789599**

(71) Applicant:
**SEIKO EPSON CORPORATION
Shinjuku-ku, Tokyo 163-0811 (JP)**

(72) Inventors:
• **Kobayashi, Michio
  Suwa-shi, Nagano-ken 392-8502 (JP)**
• **Chang, SongBin Ben
  Suwa-shi, Nagano-ken 392-8502 (JP)**

(74) Representative:
**Hoffmann, Eckart, Dipl.-Ing.
Patentanwalt,
Bahnhofstrasse 103
82166 Gräfelfing (DE)**

(54) **Method and apparatus for analyzing data**

(57) Data analysis is performed by: fundamental waveform extracting means (1) for extracting a fundamental waveform from a signal waveform of the same type as a signal under analysis; and data analysis means (2) for calculating the coefficient of correlation between the fundamental waveform and the signal under analysis while shifting the fundamental waveform by a predetermined amount along the time axis with respect to the signal under analysis, thereby analyzing the signal on the basis of the resultant correlation coefficient.

FIG. 1

EP 1 039 394 A2

**Description**

The present invention relates to a method and apparatus for analyzing data and a recording medium including a data analysis program recorded thereon, which are particularly advantageous for analysis of a periodic signal containing noise such as biological data representing, for example, heartbeats and breaths.

Two techniques are currently employed to analyze periodic signals containing noise, such as heartbeat signals and breath signals, namely the Fourier analysis and the wavelet analysis. In the Fourier analysis, the coefficient of correlation between the signal under analysis and a sine or cosine wave is calculated. Because sine and cosine waves have a continuous waveform with respect to time, the Fourier analysis is suitable for determining the correlation with a signal varying continuously and periodically. However, the Fourier analysis is not suitable for determining the correlation with a signal waveform consisting of discontinuously appearing pulses, a waveform containing a large noise component, or a waveform very different from a sine or cosine waveform.

Signals representing biological information, such as a breath signal and a heartbeat signal of a human being, unlike a sine wave, do not have a continuous waveform with respect to time, but an isolated waveform appears periodically. Besides, such biological information signals contain noise, and thus the Fourier analysis is not suitable for analyzing biological information signals.

In the wavelet analysis, on the other hand, the correlation between the waveform to be analyzed and a wavelet function is determined while shifting the wavelet function along the time axis. Therefore, this method has the advantage that a signal waveform containing discontinuous pulses can be analyzed. However, waveforms generally used in the wavelet analysis are different from the waveforms under analysis. Therefore, when the coefficient of correlation with a waveform under analysis is calculated using the wavelet analysis, the resultant correlation coefficient becomes low, and thus high-accuracy data analysis is impossible.

It an object of the present invention to provide a method and an apparatus for analyzing data, which are particularly suitable for analyzing periodic signals containing noise such as biological data signals representing, for example, heartbeats and breathings.

This object is achieved with a method as claimed in claim 1, an apparatus as claimed in claim 7 and a data storage medium as claimed in claim 13, respectively. Preferred embodiments of the invention are subject-matter of the dependent claims.

The present invention is particularly advantageous for analyzing a signal such as a heartbeat signal in which similar waveforms intermittently appear along a time axis. To this end, a waveform extracted from a signal waveform of the same type as the signal under analysis is used as a reference of fundamental waveform. The coefficient of correlation between this fundamental waveform and the signal under analysis is calculated while shifting the fundamental waveform by a predetermined amount along the time axis, and signal is analyzed on the basis of the resultant correlation coefficient.

By producing the fundamental waveform from a signal of the same type as the signal under analysis and calculating the coefficient of correlation between this fundamental waveform and the signal under analysis, it becomes possible to obtain a high correlation coefficient which allows high-accuracy data analysis.

Furthermore, by producing a waveform similar to the fundamental waveform and employing the resultant similar waveform as a new fundamental waveform, it is possible to adapt the fundamental waveform to the frequency of the signal under analysis, that is the width, along the time axis, of the signal under analysis so as to ensure high-accuracy data analysis.

When the fundamental waveform is extracted from a signal of the same type as the signal under analysis, if the fundamental waveform is modified such that equation (4) is satisfied, then the correlation coefficient can be correctly determined even when the signal under analysis is shifted upward or downward with respect to its base line. This ensures higher-accuracy data analysis.

Furthermore, when the fundamental waveform is extracted from a signal of the same type as the signal under analysis, if the fundamental waveform is normalized such that equation (5) is satisfied, the correlation coefficient can be correctly determined even when the fundamental waveform is scaled up or down along the time axis. That is, the satisfaction of equation (5) is identical to the normalization of the area of the fundamental waveform so that the coefficient of correlation with the signal under analysis can be correctly determined even when the fundamental waveform is scaled up or down along the time axis.

If both equations (4) and (5) are satisfied, a still better result can be obtained.

Furthermore, when the fundamental waveform is extracted from a signal of the same type as the signal under analysis, it is preferable that a part of the signal under analysis be normalized such that equation (6) is satisfied and then the coefficient of correlation with the fundamental waveform be calculated. By normalizing the signal under analysis in the above-described manner in addition to the fundamental waveform, it becomes possible to correctly determine the correlation coefficient regardless of the width, along the time axis, of the signal under analysis. If equations (4), (5), and (6) are all satisfied, the correlation coefficient can be determined still more accurately. This allows very accurate data analysis.

| Fig. 1 | is a schematic diagram illustrating an apparatus for analyzing data according to the present invention. |
|---|---|

Fig. 2 is a schematic diagram illustrating the process of extracting a fundamental waveform from a signal of the same type as a signal under analysis and determining the coefficient of correlation between the fundamental waveform and the signal under analysis.

Fig. 3 is a schematic diagram illustrating the positional relationship between a fundamental waveform and a signal under analysis in the process of determining the coefficient of correlation between them.

Fig. 4 is a schematic diagram illustrating the process of calculating the coefficient of correlation between a fundamental waveform and a signal under analysis wherein the fundamental waveform and the signal under analysis have the same shape.

Fig. 5 is a schematic diagram illustrating the process of calculating the coefficient of correlation between a fundamental waveform and a signal under analysis wherein the fundamental waveform and the signal under analysis are different in shape.

Fig. 6 is a schematic diagram illustrating the process of calculating the coefficient of correlation between a fundamental waveform and a signal under analysis wherein the fundamental waveform and the signal under analysis have the same shape, and the fundamental waveform is modified so that equation (4) is satisfied.

Fig. 7 is a schematic diagram illustrating the process of calculating the coefficient of correlation between a fundamental waveform and a signal under analysis wherein the fundamental waveform and the signal under analysis are different in shape, and the fundamental waveform is modified so that equation (4) is satisfied.

Fig. 8 is a schematic diagram illustrating the process of calculating the coefficient of correlation between a fundamental waveform and a signal under analysis wherein the fundamental waveform and the signal under analysis have the same shape.

Fig. 9 is a schematic diagram illustrating the process of calculating the coefficient of correlation between the fundamental waveform and the signal under analysis shown in Fig. 8, wherein the frequency of the fundamental waveform is varied from that shown in Fig. 8 but normalization of the fundamental waveform is not performed.

Fig. 10 is a schematic diagram illustrating the process of calculating the coefficient of correlation between a fundamental waveform and a signal under analysis wherein the fundamental waveform and the signal under analysis have the same shape.

Fig. 11 is a schematic diagram illustrating the process of calculating the coefficient of correlation between the fundamental waveform and the signal under analysis shown in Fig. 8, wherein the frequency of the fundamental waveform is varied from that shown in Fig. 8, and normalization is performed for both the fundamental waveform and the signal under analysis.

Fig. 12 illustrates, in the form of contour lines, a correlation coefficient calculated for a signal under analysis containing no noise according to the present invention, and also illustrates a result obtained by performing a Fourier analysis upon the correlation coefficient.

Fig. 13 illustrates, in the form of contour lines, correlation coefficients calculated for a signal under analysis containing noise according to the present invention, and also illustrates a result obtained by performing a Fourier analysis upon the correlation coefficients.

Fig. 14 illustrates a result obtained by performing a Fourier analysis upon correlation coefficients calculated according to the present invention, and also illustrates, for the purpose of comparison, a result obtained only by performing a Fourier analysis according to a conventional technique.

Fig. 15 illustrates an example of a signal under analysis that contains noise, wherein the results shown in Fig. 14 are obtained for the signal shown herein in Fig. 15.

Fig. 16 illustrates an example of a heartbeat signal waveform and examples of fundamental waveforms extracted from this heartbeat signal waveform, wherein one example of the fundamental waveform includes two dips and the other includes two peaks.

Fig. 17     illustrates an example of a heartbeat signal having a missing heartbeat and having a fluctuation in period.

Fig. 18     illustrates, in the form of contour lines, the correlation coefficient obtained by performing the process upon the heartbeat signal shown in Fig. 17 according to the present invention.

Embodiments of the present invention are described below. In the embodiments described below, specific examples of the method and apparatus for analyzing data according to the present invention are described below. It should be understood that the specific examples described below may also be applied to the data analysis program recorded on the recording medium according to the present invention.

The method of analyzing data according to the present invention is advantageous in particular when it is used to analyze biological information such as breaths and heartbeats. In many cases, biological information is given by a signal in which similar waveforms intermittently appear along a time axis. One technique of analyzing such a signal waveform is to calculate the coefficient of correlation of an input signal (that is, a signal to be analyzed) with respect to a fundamental signal waveform (fundamental or reference waveform) and perform data analysis on the basis of the resultant correlation coefficient.

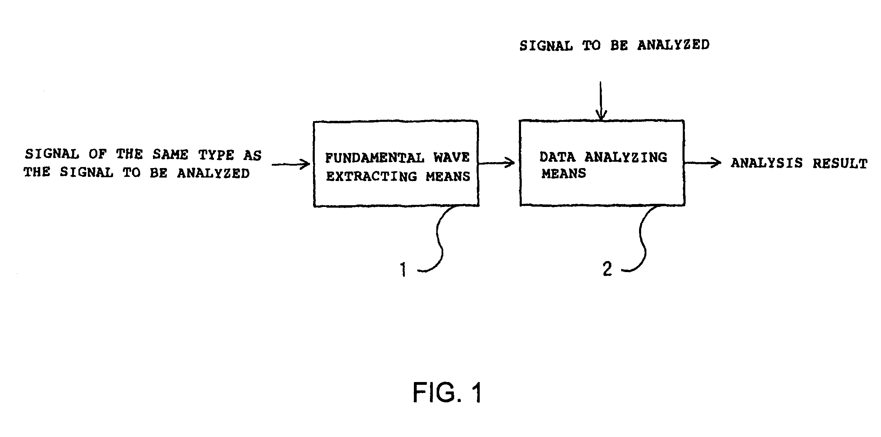

The present invention is characterized in that a reference or fundamental waveform is produced from a waveform of a signal of the same type as a signal under analysis. Fig. 1 is a schematic diagram illustrating an apparatus for analyzing data according to the present invention. The apparatus includes a fundamental waveform extracting means 1 for extracting a fundamental waveform from a signal waveform of the same type as a signal under analysis; and data analyzing means 2 for calculating the coefficient of correlation between the extracted fundamental waveform and the signal under analysis while shifting the fundamental waveform by a predetermined amount along the time axis, and analyzing the signal on the basis of the obtained correlation coefficient.

For example, when a signal is analyzed using the data analyzing apparatus constructed in the above-described manner, if the signal under analysis is a signal corresponding to heartbeats (hereinafter referred to as a heartbeat signal), a part of a particular heartbeat signal is employed as a fundamental waveform. More specifically, a heartbeat signal containing no noise is acquired in a normal state (e.g., from a person under no-load condition), and a fundamental waveform is produced by extracting a part of the heartbeat signal. The correlation coefficient between a heartbeat signal being actually analyzed (which often contains noise or irregularities) and the fundamental waveform is calculated while shifting the fundamental waveform along a time axis. That is, the fundamental waveform is approximated by a certain function, and the correlation coefficient is calculated between the signal under analysis and the approximation function. Herein, a wavelet function is employed as the function approximating the fundamental waveform.

To obtain various kinds of information such as a heart rate or the state of heartbeats, data analysis is then performed on the basis of the calculated coefficient of correlation between the fundamental waveform and the signal under analysis.

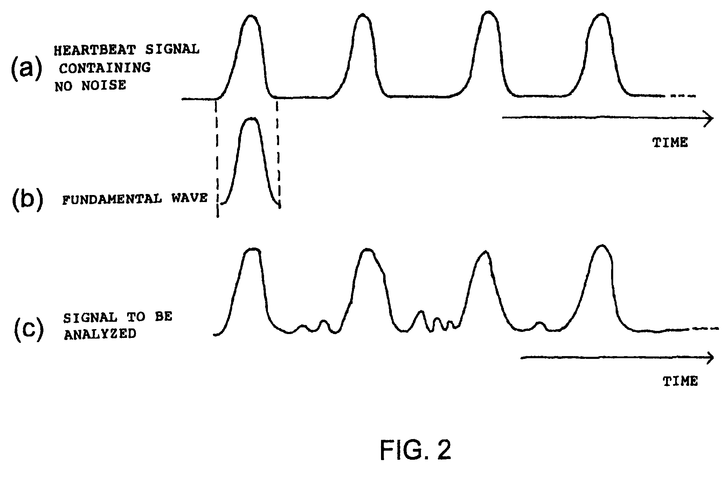

The above process is described in further detail below with reference to Fig. 2. Fig. 2(a) illustrates an example of a heartbeat signal obtained in a normal state and containing no noise. From this heartbeat signal, a fundamental waveform is obtained as shown in Fig. 2(b). The coefficient of correlation between the heartbeat signal under analysis (signal under analysis) and the fundamental waveform is calculated while shifting the fundamental waveform in the time axis. On the basis of the resultant correlation coefficient, various kinds of information such as the heart rate can be obtained. The correlation coefficient may be basically determined using the following equation:

$$C = \int_{-\infty}^{\infty} s(t) \cdot h(t) dt \qquad (1)$$

In equation (1), s(t) is the signal under analysis, h(t) is the fundamental waveform, and C is the correlation coefficient to be determined. When the fundamental waveform is shifted along the time axis, if the time shift is denoted by b, then equation (1) described above can be represented as follows.

$$C = \int_{-\infty}^{\infty} s(t) \cdot h(t - b) dt \qquad (2)$$

When the waveform of the signal under analysis is identical to the fundamental waveform, the correlation coefficient C has its maximum value 1. Generally $C \leq 1$ holds true, i.e., the correlation coefficient C varies within the range $0 \leq C \leq 1$ depending on the degree of correlation between the signal under analysis and the fundamental waveform.

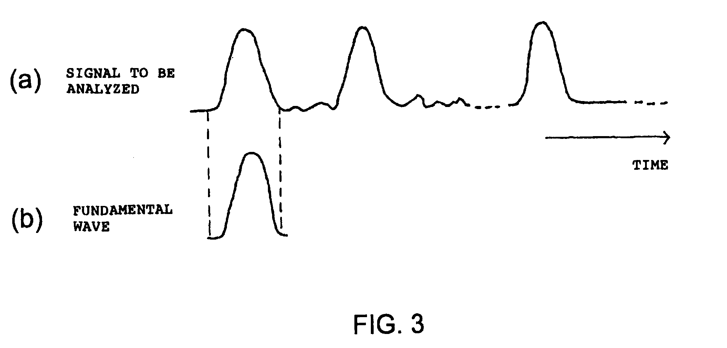

Now, let us assume that the signal under analysis and the fundamental waveform have a relative temporal relationship such as shown in Figs. 3(a) and 3(b). In this situation, the correlation coefficient C can be determined in accordance with equation (1) (herein, for the sake of simplicity, the time shift b of the fundamental waveform along the time axis is assumed to be b=0). That is, the correlation coefficient is obtained by multiplying the signal s(t) under analysis by the fundamental waveform h(t) and integrating the resultant product over the range equal to the length of the fundamental waveform.

Herein, if the signal under analysis is identical in shape to the fundamental waveform, the correlation coefficient C becomes C = 1. However, if the signal under analysis is different in shape from the fundamental waveform, the correlation coefficient C obtained by integrating the product of the signal under analysis and the fundamental waveform over the range equal to the length of the fundamental waveform should have a value smaller than 1.

In the present invention, as described above, the fundamental waveform is produced from a signal waveform of the same type as the signal under analysis, that is a normal heartbeat signal containing no noise when the signal under analysis is a heartbeat signal, and the coefficient of correlation between the fundamental waveform and the signal under analysis is calculated. Thus, it is possible to obtain a high correlation coefficient which allows data analysis to be performed in an accurate manner.

When the fundamental signal is produced from the signal of the same type of the signal under analysis, and the correlation between the fundamental waveform and the signal under analysis is determined, preferred embodiments of the present invention impose some constraints as described below.

As mentioned before, in the case where the heart rate is to be measured, a fundamental (reference) waveform is produced from a normal heartbeat signal waveform, and data analysis is performed upon the signal under analysis (heartbeat signal under analysis) using the produced fundamental waveform. In this case, the shape the produced fundamental waveform can be assumed to be similar to the shape of the actual heartbeat signal waveform under analysis even though there can be a difference in frequency, or, more precisely, in the time scale. It is thus, preferable to change the "frequency" (time scale) of the fundamental waveform depending on the frequency of the signal under analysis such that the resultant fundamental waveform with the modified "frequency" has the closest resemblance to the signal under analysis.

To achieve this, first, a waveform similar to the fundamental waveform is produced by varying the frequency of the fundamental waveform. More specifically, the fundamental waveform represented by h(t) is converted to a similar waveform h(t/a) where the time scaling factor a is an arbitrary value. By properly selecting the value of the scaling factor a, it is possible to compress or expand the fundamental waveform along the time axis. The value of the scaling factor a is set to be optimum depending on the frequency of the signal under analysis. After producing a waveform similar to the fundamental waveform depending on the frequency of the signal under analysis, the resultant similar waveform is employed as a new fundamental waveform, and the coefficient of correlation between the fundamental waveform and the signal under analysis is calculated. When a fundamental waveform similar to the original fundamental waveform is employed, equation (2) becomes:

$$C = \int_{-\infty}^{\infty} s(t)\{h(t - b)/a\}dt \qquad (3)$$

In accordance with preferred embodiments of the present invention the accuracy in obtaining the coefficient of correlation can be further increased, if the fundamental waveform is produced such that one or more of the following equations (4) to (6) is/are satisfied:

$$\int_{-\infty}^{\infty} h(t)dt = 0 \qquad (4)$$

$$\int_{-\infty}^{\infty} |h(t)|^2 dt = 1 \qquad (5)$$

$$\int_{j}^{k} |s(t)|^2 dt = 1 \qquad (6)$$

Equation (4) requires that the base line of the fundamental waveform be set such that the fundamental waveform has a positive part and a negative part with respect the base line balanced such that the integral of the fundamental waveform over its length becomes equal to 0. When, under this condition, the correlation coefficient C is calculated in accordance with equation (3), the same result is obtained at whatever location relative to the base line the signal under analysis is.

For example, we assume that the signal under analysis is shifted by y in the positive direction, that is, s(t) is modified to s(t) + y. If equation (1) is employed for the sake of simplicity, the correlation coefficient is calculated as follows:

$$C = \int_{-\infty}^{\infty} \{s(t) + y\} \cdot h(t)dt \qquad (7)$$

$$= \int_{-\infty}^{\infty} s(t) \cdot h(t)dt + y \int_{-\infty}^{\infty} h(t)dt$$

If equation (4) representing the above condition is substituted into equation (7), then equation (7) becomes as follows:

$$C = \int_{-\infty}^{\infty} s(t) \cdot h(t)dt \qquad (8)$$

Thus, the resultant equation (8) becomes identical to equation (1). In the case where the signal under analysis is shifted by y in the negative direction, that is, s(t) is modified to s(t) - y, the same correlation coefficient is obtained. This means that the correct correlation coefficient is obtained regardless of the position of the signal under analysis with respect to the base line.

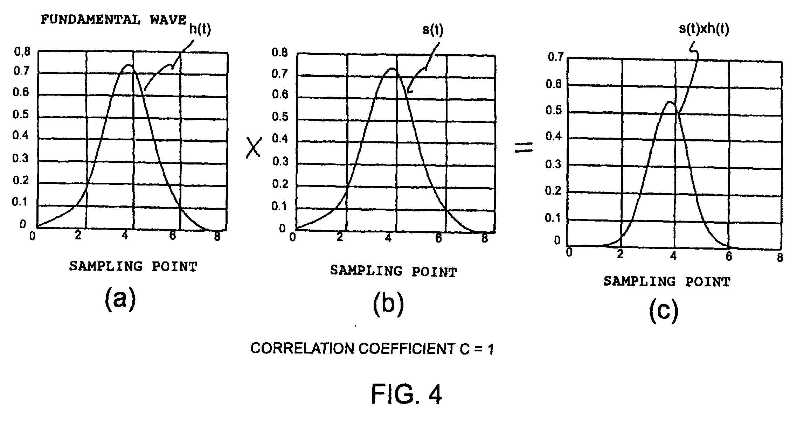

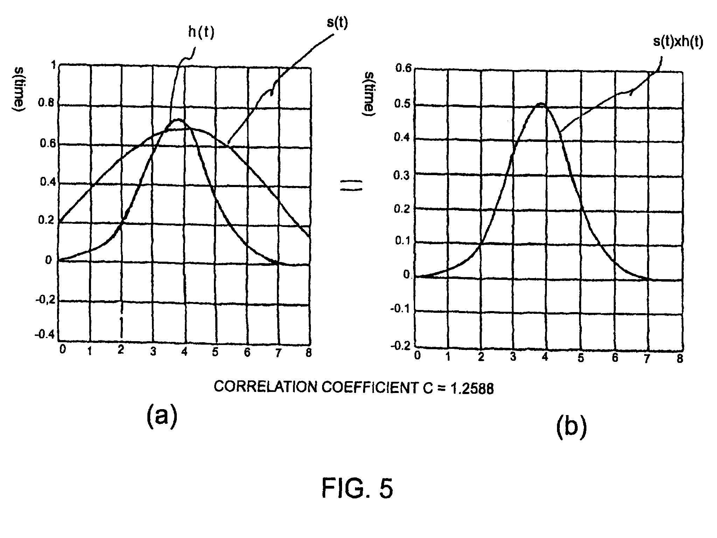

The above process is described in further detail below with reference to specific examples. Figs. 4 and 5 illustrate examples in which the correlation coefficient C is calculated using fundamental waveforms which do not satisfy equation(4). In the example shown in Fig. 4, the fundamental waveform has the same shape as the signal under analysis. However, the shape of the fundamental waveform is very different from that of the signal under analysis in the example shown in Fig. 5.

Fig. 4(a) illustrates a fundamental waveform which does not satisfy equation (4), and Fig. 4(b) illustrates a signal being analyzed and having the same shape as the fundamental waveform. If the signal under analysis shown in Fig. 4(b) is multiplied by the fundamental waveform shown in Fig. 4(a), the resultant waveform becomes as shown in Fig. 4(c). The correlation coefficient C obtained by integrating the product over the range equal to the length of the fundamental waveform according to equation (1) becomes C = 1, because these two waveforms have the same shape.

On the other hand, as shown in Fig. 5(a), if a signal s(t) under analysis is multiplied by a fundamental waveform h(t) which does not satisfy equation (4), the resultant signal waveform becomes as shown in Fig. 5(b). In this case, the correlation coefficient C obtained by integrating the product over the range equal to the length of the fundamental waveform according to equation (1) becomes C = 1.2588. In this example shown in Fig. 5, the correlation coefficient C should be less than 1, because the signal under analysis and the fundamental waveform are very different in shape from each other. However, the correlation coefficient C = 1.2588 actually obtained is greater than 1.

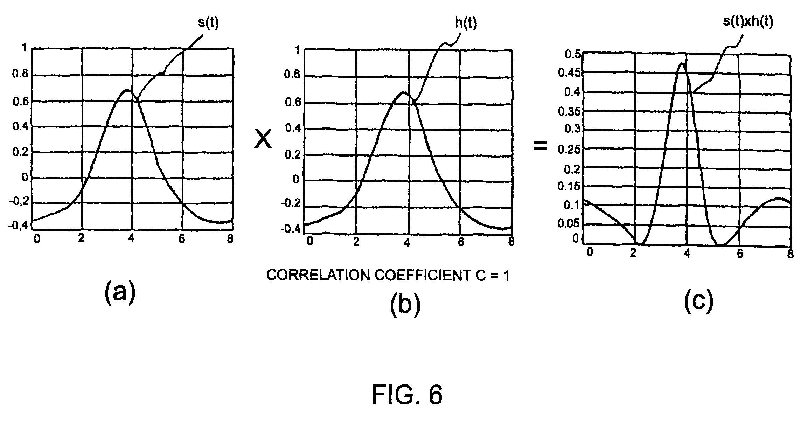

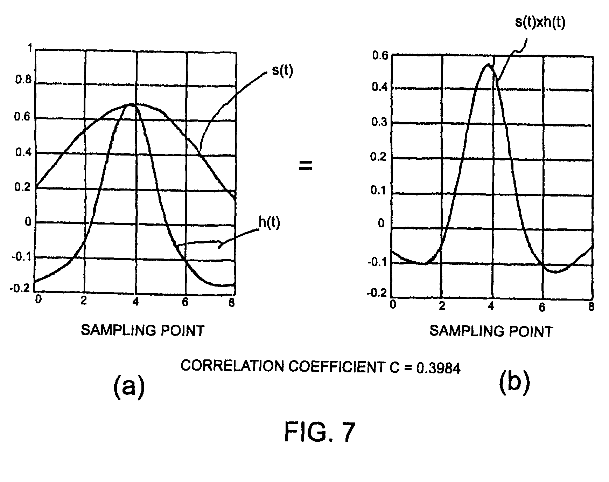

Figs. 6 and 7 show examples in which the correlation coefficient C is calculated using fundamental waveforms which satisfy equation (4). Fig. 6(a) illustrates a fundamental waveform which satisfies equation (4), and Fig. 6(b) illustrates a signal being analyzed and having the same shape as the fundamental waveform. If the signal under analysis shown in Fig. 6(b) is multiplied by the fundamental waveform shown in Fig. 6(a), the resultant waveform becomes as shown in Fig. 6(c). The correlation coefficient C obtained by integrating the product over the length of the fundamental waveform according to equation (1) becomes C = 1, because these two waveforms have the same shape.

On the other hand, as shown in Fig. 7(a), if a signal s(t) under analysis is multiplied by a fundamental waveform h(t) which satisfies equation (4), the resultant signal waveform becomes as shown in Fig. 7(b). In this case, the correlation coefficient C obtained by integrating the product over the length of the fundamental waveform according to equation (1) becomes C = 0.3984. In this example shown in Fig. 7, the correlation coefficient C should be less than 1, because the signal under analysis and the fundamental waveform are very different in shape from each other. The obtained calculation result C = 0.3984 satisfies this requirement.

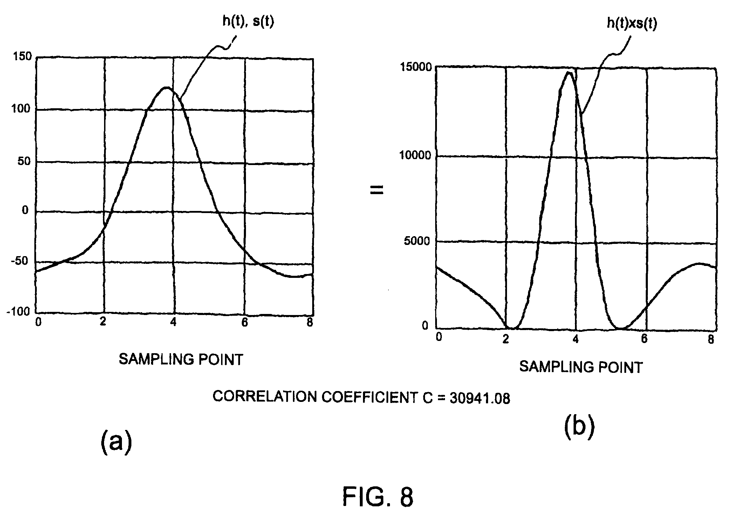

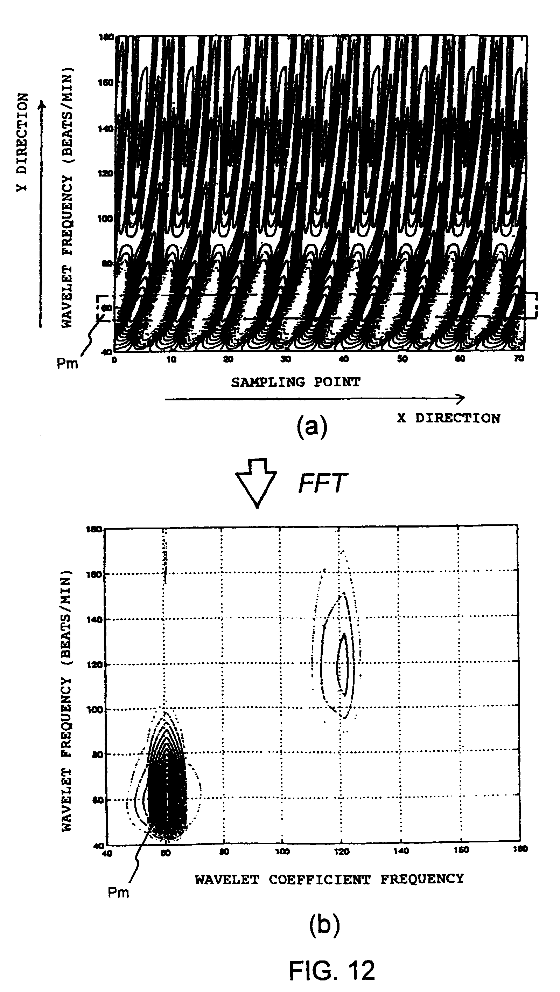

In Figs. 4 to 7, the time axis is taken in the horizontal direction, and numerals on the time axis represent sampling points. The time axis and sampling points are represented in a similar manner also in Figs. 8 to 18 which will be referred later, except for Figs. 12(b), 13(b), and 14(b) which illustrate results obtained via Fourier analysis.

The advantage achieved when the fundamental waveform is produced such that equation (5) is satisfied, is explained below.

As described above, the fundamental waveform may be scaled up (expanded) or down (compressed) along the time axis so as to obtain a waveform similar to the signal under analysis. However, if the scaled-up or scaled-down fundamental waveform is directly employed, a good result cannot be obtained. For example, let us assume that we have a signal s(t) under analysis and a fundamental waveform h(t) having both having the same shape, as shown in Fig. 8(a). Because the fundamental waveform h(t) and the signal s(t) under analysis have the same shape, the coefficient of correlation between these waveforms should have the maximum possible value. We assume, in this example, that the product of the fundamental waveform h(t) and the signal s(t) under analysis, that is h(t) × s(t), becomes as shown in Fig. 8(b), and that the correlation coefficient C obtained by integrating the product over the length of the fundamental waveform is C = 30941.08.

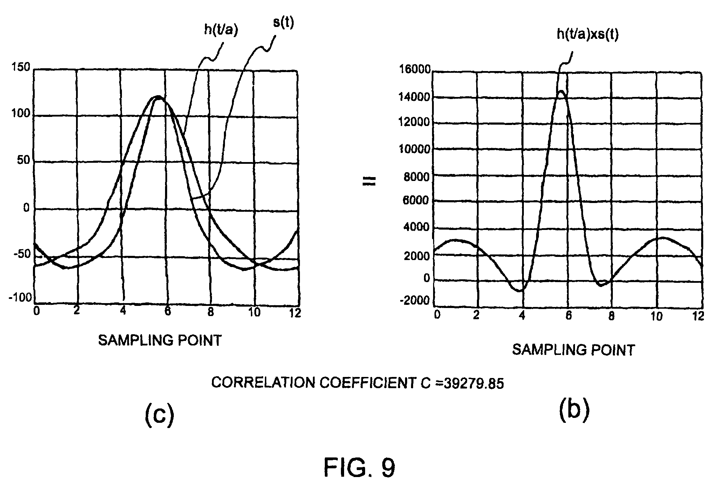

Herein, if the fundamental waveform is modified to h(t/a), that is, expanded along the time axis as shown in Fig. 9(a), and if the coefficient of correlation between the modified fundamental waveform h(t/a) and the same signal s(t) under analysis as that shown in Fig. 8(a) is calculated in accordance with equation (1), then the product of the fundamental waveform h(t/a) and the signal s(t) under analysis, that is h(t) × s(t) becomes as shown in Fig. 9(b), and the correlation coefficient C obtained by integrating the product over the length of the fundamental waveform becomes C = 39279.85.

The correct correlation coefficient for the example shown in Fig. 9 should be smaller than that for the example shown in Fig. 8. However, the calculated value for the example shown in Fig. 9 is greater than that for the example shown in Fig. 8. This problem is due to the difference in the area (power) of the fundamental waveform.

According to equation (5) this problem can be avoided, by normalizing the area of the fundamental waveform so as to ensure that the correlation coefficient for the same signal under analysis can be correctly calculated no matter how the time scaling factor a in the fundamental waveform is varied. A practical method for the normalization is to multiply the fundamental waveform by a constant so that the area calculated by integrating the square of the resultant fundamental waveform always becomes 1 no matter how the time scaling factor for the fundamental waveform is chosen. For example, when the fundamental waveform is scaled down along the time axis, the fundamental waveform is expanded in a vertical direction by multiplying the fundamental waveform by a constant thereby increasing the area thereof.

Note that Figures 8 and 9 show examples that satisfy equation (4) explained above. While equations (4) and (5) need not necessarily be employed together, the result obtained when the fundamental waveform is generated so as to satisfy both, equation (4) and (5), is even better than with each of equations (4) and (5) alone.

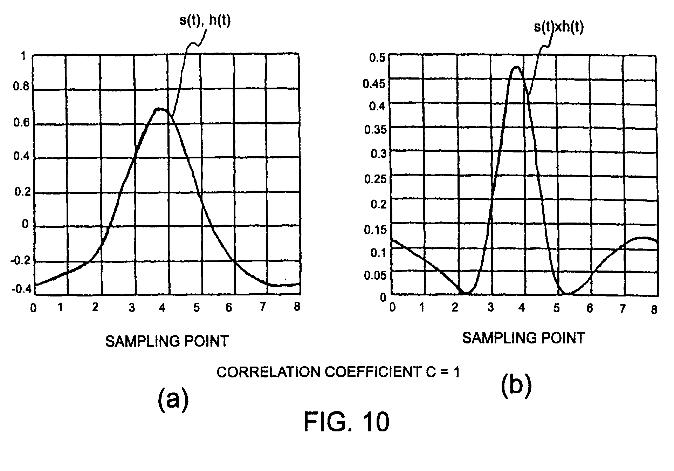

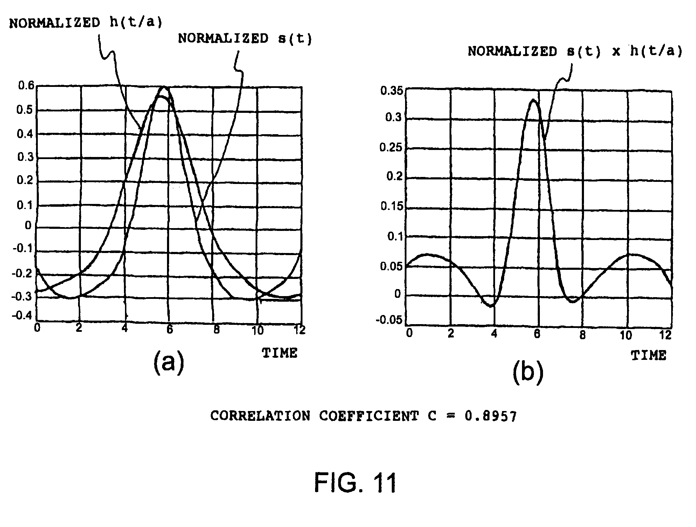

The accuracy in obtaining the coefficient of correlation is also increased, when part of the signal under analysis is normalized in accordance with equation (6), and then the correlation coefficient is calculated for the resultant normalized signal under analysis. By normalizing the signal under analysis in addition to the fundamental waveform, it becomes possible to calculate the correlation coefficient more accurately. Figs. 10 and 11 illustrate examples in which a correlation coefficient is calculated after normalizing both a fundamental waveform and a signal under analysis.

When a correlation coefficient is calculated after normalizing both a fundamental waveform h(t) and a signal s(t) under analysis, if the fundamental waveform h(t) and the signal s(t) under analysis are identical to each other as shown in Fig. 10(a), the product of the fundamental waveform and the signal under analysis, that is h(t) × s(t) becomes as shown in Fig. 10(b), and the correlation coefficient obtained by integrating the product over the length of the fundamental waveform becomes C = 1.

Now we assume that the fundamental waveform is converted to h(t/a), that is, expanded along the time axis. When the coefficient of correlation between the modified fundamental waveform h(t/a) and the same signal s(t) under analysis as that shown in Fig. 10(a) is calculated in accordance with equation (1), if the fundamental waveform h(t/a) is normalized according to equation (5) and the signal s(t) under analysis is normalized according to equation (6), then the resultant normalized fundamental waveform and signal under analysis become, for example, as shown in Fig. 11(a).

The product of the normalized fundamental waveform h(t/a) and the normalized signal s(t) under analysis, that is h(t/a) × s(t) becomes as shown in Fig. 11(b). If the correlation coefficient C is determined by integrating the product over the length of the fundamental waveform, then a correct value is obtained as C = 0.8957.

Thus, information as to the signal under analysis can be obtained by calculating the correlation coefficient in the above-described manner. More specifically, in the case of the present embodiment in which a heartbeat signal is given as the signal under analysis, information about the heartbeats (such as the heart rate) is obtained. Fig. 12 illustrates an example in which a correlation coefficient is obtained for a signal containing substantially no noise.

Fig. 12(a) illustrates a three-dimensional spatial coordinate system consisting of x-, y-, and z-axes, projected onto a plane (x-y plane). Sampling points along the time axis are taken in the x direction, and heart rates (beats/min) are taken in the y axis. Furthermore, correlation coefficients are represented by contour lines as measured in the z axis (not shown) perpendicular to the x-y plane. Highest contour lines (peaks) are indicated by Pm. In the present embodiment, if the value representing a particular sampling point in the x axis is multiplied by 1/8, then a corresponding time in units of seconds is obtained. For example, a sampling point 40 corresponds to 5 s in the time axis.

In the example in Fig. 12(a), the correlation coefficient becomes highest at points where the heart rate is equal to 60 beats/min, for various sampling points. Thus, it can be determined that the signal under analysis (heartbeat signal) has a frequency of 60 beats/min.

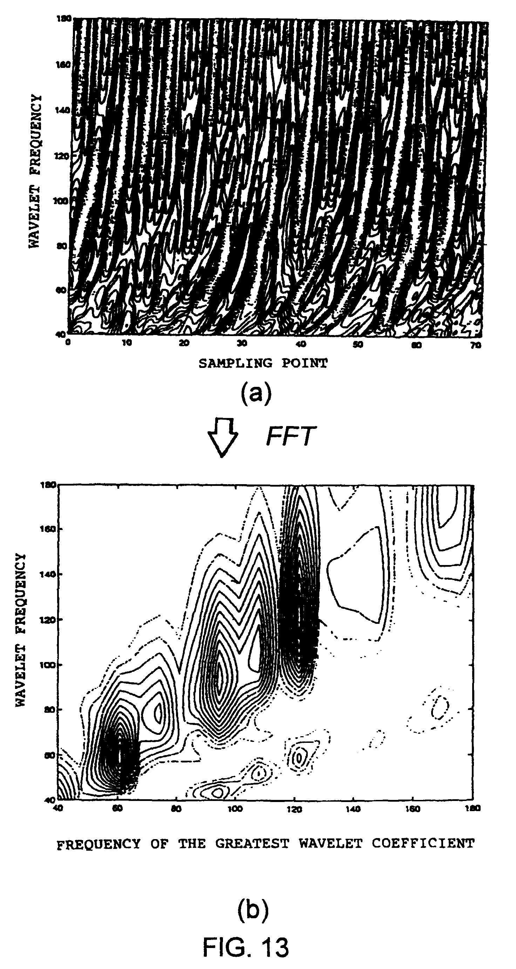

In the example shown in Fig. 12(a), because the signal under analysis contains substantially no noise, heart rates at respective sampling points are clearly indicated by determining correlation coefficients. However, as shown in Fig. 13(a), when the signal under analysis contains large noise components, there are no clear peaks of the contour lines, and thus it is impossible to definitely determine the heart rate.

In an embodiment of the present invention, to avoid the above problem, a Fast Fourier analysis (FFT) is applied to the above result. For example, if a Fourier analysis is performed upon the result shown in Fig. 12(a), then the result becomes as shown in Fig. 12(b). On the other hand, if a Fourier analysis is performed upon the result shown in Fig. 13(a), the result becomes as shown in Fig. 13(b). Although in the example shown in Fig. 12(a) a good result is obtained without performing the Fourier analysis, the Fourier analysis allows the peak Pm of the contour lines to be obtained more clearly. That is, in this specific example, the result of the Fourier analysis clearly indicates that the heart rate is 60 beats/min. In the example of Fig. 13(a) in which no clear peaks are obtained and thus the heart rate cannot be definitely determined, the result of the Fourier analysis, as shown in Fig. 13(b), clearly indicates a peak Pm of the contour lines. Thus, the heart rate can be definitely determined to be 60 beats/min.

As described above, by performing a Fourier analysis upon the correlation coefficients obtained in the manner described earlier, it becomes possible to obtain desired information (heart rate, in this specific example) more clearly.

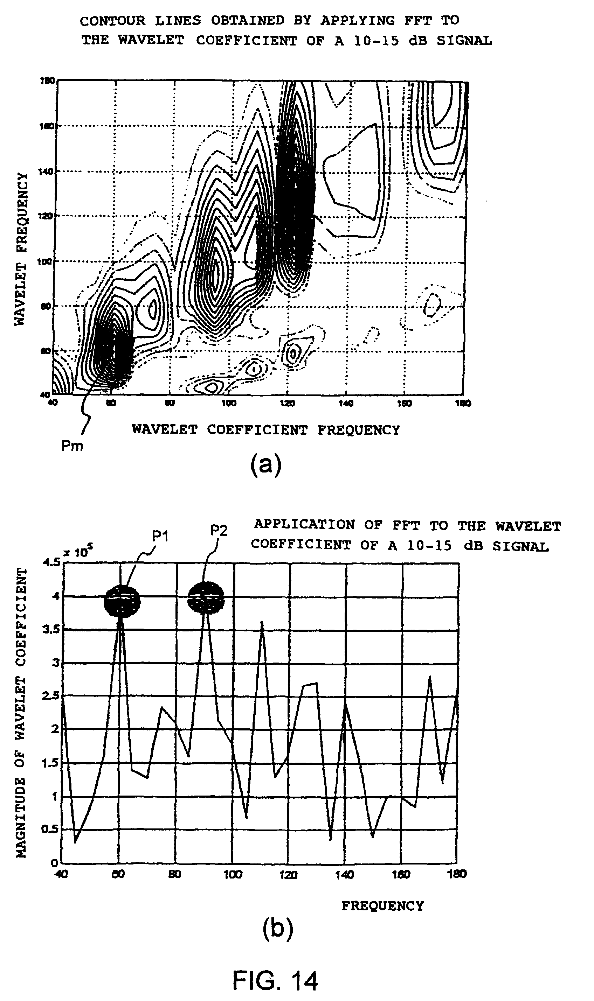

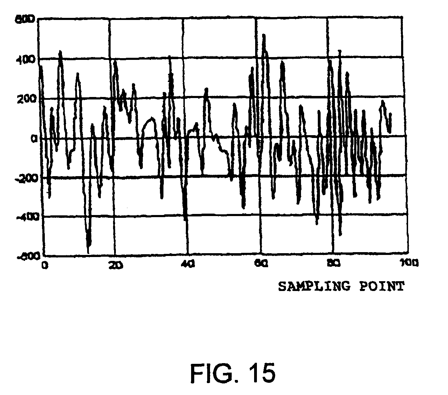

In Fig. 14, the determination of the heart rate by means of performing a Fourier analysis upon correlation coefficients in accordance with the present invention is compared with the determination by means of only a Fourier analysis according to a conventional technique, wherein the signal analyzed by both techniques contains noise (-10.15 dB) as shown in Fig. 15. Fig. 14(a) illustrates the result obtained by performing a Fourier analysis upon the correlation coefficients according to the present invention. Fig. 14(b) illustrates the result obtained by performing only a Fourier analysis according to the conventional technique.

In the result obtained by performing only a Fourier analysis, two peaks P1 and P2 appear at heart rates of 60 beats/min and 90 beats/min, and it cannot be determined which peak indicates the correct heart rate. On the other hand, in the result according to present invention, a peak Pm of the correlation coefficient appears at 60 beats/min, and thus it is possible to definitely determine that the heart rate is 60 beats/min.

In the above-described embodiments according to the present invention, when a fundamental waveform is extracted from, for example, a heartbeat signal, a waveform in a particular interval containing only a single pulse is extracted from the heartbeat signal including a large number of pulses which appear periodically along the time axis, and the extracted waveform is employed as the fundamental waveform. Alternatively, a waveform in an interval including a plurality of pulses may be employed as the fundamental waveform.

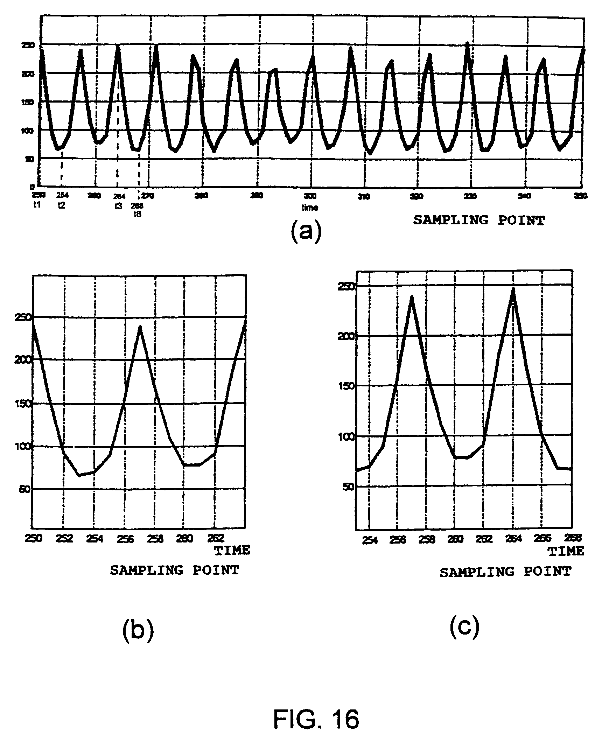

For example, when a heartbeat signal such as that shown in Fig. 16(a) is given, the waveform in the interval from time t1 (sampling point 250) to time t3 (sampling point 264) including two dips (referred to as a double dip waveform) may be extracted and employed as the fundamental waveform. Instead, a waveform in the interval from time t2 (sampling point 254) to time t4 (sampling point 268) including two peaks (referred to as a double peak waveform) may be extracted and employed as the fundamental waveform. Fig. 16(b) illustrates the double dip waveform, and Fig. 16(c) illustrates the double peak waveform, which are extracted in the above-described manner.

Also in the case where a double dip waveform or a double peak waveform is employed as the fundamental waveform, a good result can be obtained if equations (5) and (6) are satisfied. An experiment has been performed to determine which of the fundamental waveforms, the double dip waveform or the double peak waveform, is more suitable for detecting the heart rate. The experiment has revealed that a better result can be obtained when a double dip waveform is employed as the fundamental waveform than can be obtained when a double peak waveform is employed.

In the embodiments of the present invention, as described above, when a heartbeat signal of someone is analyzed, a signal in a particular interval is extracted from a heartbeat signal, and the extracted waveform is employed as a fundamental waveform. The coefficient of correlation between this fundamental waveform and the signal under analysis is calculated while shifting the fundamental waveform along the time axis with respect to the signal under analysis. Data analysis is then performed upon the signal on the basis of the obtained correlation coefficient so as to obtain information such as a heart rate.

Because the fundamental waveform is obtained from the signal of the same type as the signal under analysis, it is possible to obtain a higher correlation coefficient than is obtained by the conventional technique in which a wavelet function is employed without taking account the signal under analysis. Thus, it is possible to perform data analysis in a more accurate manner.

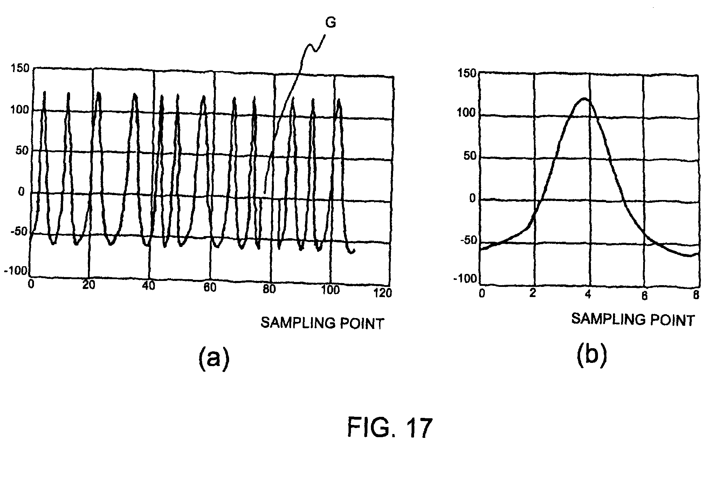

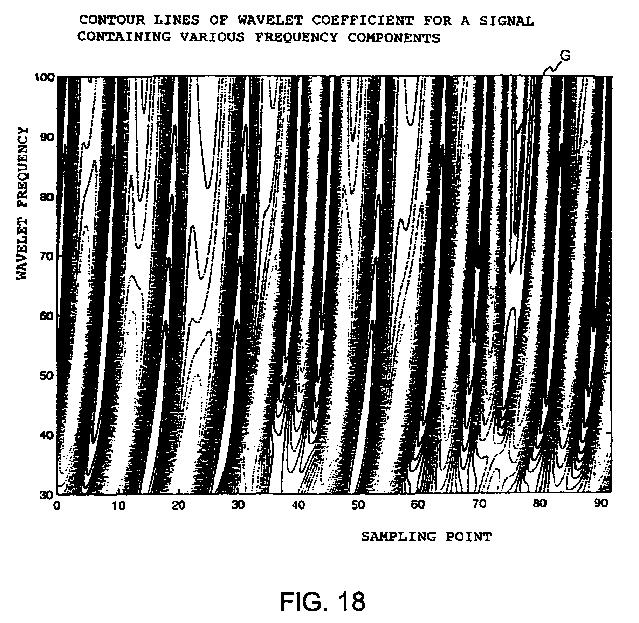

When there is a missing heartbeat G in a heartbeat signal of someone as shown in Fig. 17(a), if the coefficient of correlation with a fundamental waveform shown in Fig. 17(b) is calculated, and if data analysis is performed on the basis of the obtained correlation coefficient, then the result becomes as shown in Fig. 18. In addition to the missing heartbeat, the heartbeat signal shown in Fig. 17(a) has a variation in the heartbeat period. The variation in the period and the missing heartbeat can also be detected from Fig. 18. More specifically, in Fig. 17(a), the interval from sampling point 0 to about 40 has a relatively long period, and an interval from sampling point 40 to 50 has a relatively short period. The interval from sampling point 50 to 60 has a relatively long period again. After sampling point 65, the period again decreases. There is a missing heartbeat G in the interval from sampling point 70 to 80. These variations in the period and the missing heartbeat can also be detected from Fig. 18.

The present invention is not limited to the embodiments descried above, but various modifications are possible without departing from the scope of the invention as defined by the appended claims. For example, as for the wavelet function employed in the present invention to approximate the fundamental waveform, there are various types of wavelet functions which can be employed in the invention. They include an orthogonal wavelet function, a biorthogonal wavelet function, a spline wavelet function, a Daubechies wavelet function, a Symlet wavelet function, a Coiflet wavelet function, a non-orthogonal wavelet function, and a wavelet packet function.

As for the fundamental waveform, in addition to the shapes described above, a waveform including a single dip or a waveform including three peaks or dips may also be employed.

The apparatus and method of the present invention in its various embodiments described above can be implemented by means of a computer program loaded and executed in a general-purpose computer. Such computer program may be stored on a machine-readable storage medium such as a floppy disk, an optical disk, a hard disk, a semiconductor memory, etc. The program may also be loaded via a computer network.

**Claims**

1.  A method of analyzing a signal in which similar waveforms intermittently appear along a time axis, comprising:

    (a) preparing a fundamental waveform;
    (b) calculating the coefficient of correlation between said fundamental waveform and the signal under analysis; and
    (c) obtaining information contained in the signal under analysis,
    **characterized in that**

    step (a) comprises extracting said fundamental waveform from a signal waveform of the same type as said signal under analysis;
    step (b) comprises calculating the coefficient of correlation between said fundamental waveform and said signal under analysis while shifting said fundamental waveform by a predetermined amount along the time axis with respect to said signal under analysis; and
    step (c) comprises said signal is analyzed on the basis of the resultant correlation coefficient.

2.  The method according to Claim 1, wherein step (a) comprises

    (a1) extracting a waveform in a particular interval along the time axis from the signal waveform of the same type as said signal under analysis, and
    (a2) approximating said extracted waveform by a function h(t), and then producing a similar waveform h(t/a) from said function h(t), wherein a is an arbitrary value, and employing the similar waveform as said fundamental waveform.

3.  The method according to Claim 2, wherein a wavelet function is employed as said function h(t) in step (a2).

4.  The method according to any one of Claims 1 to 3, wherein step (a) comprises modifying the extracted fundamental waveform such that the following

equation is satisfied:

$$\int_{-\infty}^{\infty} h(t)dt = 0 \qquad (4)$$

where h(t) is the modified fundamental waveform.

5.  The method according to any one of Claims 1 to 4, wherein step (a) comprises normalizing said extracted fundamental waveform such that the following equation is satisfied:

$$\int_{-\infty}^{\infty} |h(t)|^2 dt = 1 \qquad (5)$$

where h(t) is the normalized fundamental waveform.

6.  The method according to any one of Claims 2 to 5, wherein step (b) comprises normalizing a part of said signal under analysis such that the following equation is satisfied:

$$\int_{j}^{k} |s(t)|^2 dt = 1 \qquad (6)$$

where s(t) is said part of said signal under analysis, and j and k represent the start and end points of said interval along the time axis; and

    after completion of the normalization, the coefficient of correlation with said fundamental waveform is calculated.

7.  An apparatus for analyzing a signal in which similar waveforms intermittently appear along a time axis, comprising

    waveform generating means (1) for preparing a fundamental waveform, and
    data analyzing means (2) for calculating the coefficient of correlation between said fundamental waveform and said signal under analysis and for obtaining information contained in the signal under analysis,
    **characterized in that**
    said waveform generating means (1) is means for extracting said fundamental waveform from a signal waveform of the same type as said signal under analysis; and
    said data analysis means is adapted to calculate the coefficient of correlation between said fundamental waveform and said signal under analysis while shifting said fundamental waveform by a predetermined amount along the time axis with respect to said signal under analysis, thereby analyzing said signal on the basis of the resultant correlation coefficient.

8. The apparatus according to Claim 7, wherein said waveform generating means (1) comprises:

means for extracting a waveform in a particular interval along the time axis from the signal waveform of the same type as said signal under analysis, and
means for approximating said extracted waveform by a function hit), and then producing a similar waveform h(t/a) from said function h(t), wherein a is an arbitrary value, and employing the similar waveform as said fundamental waveform.

9. The apparatus according to Claim 8, wherein a wavelet function is employed as said function h(t).

10. The apparatus according to any one of Claims 7 or 9, wherein said waveform generating means (1) comprises:

means for modifying the extracted fundamental waveform such that the following equation is satisfied:

$$\int_{-\infty}^{\infty} h(t)dt = 0 \qquad (4)$$

where h(t) is the modified fundamental waveform.

11. The apparatus according to any one of Claims 7 or 10, wherein said waveform generating means (1) comprises:

means for normalizing said extracted fundamental waveform such that the following equation is satisfied:

$$\int_{-\infty}^{\infty} |h(t)|^2 dt = 1 \qquad (5)$$

where h(t) is the normalized fundamental waveform.

12. The apparatus according to one of Claims 8 or 11, wherein said waveform generating means (1) comprises:

means for normalizing a part of said signal under analysis such that the following equation is satisfied:

$$\int_{j}^{k} |s(t)|^2 dt = 1 \qquad (6)$$

where s(t) is said part of said signal under analysis, and j and k represent the start and end points of said interval along the time axis; and
means for calculating, after completion of the normalization, the coefficient of correlation with said fundamental waveform.

13. A machine-readable data storage medium carrying computer program means that when executed in an apparatus as defined in any one of claims 7 to 12, carries out a method as defined in any one of claims 1 to 6.

SIGNAL TO BE ANALYZED

SIGNAL OF THE SAME TYPE AS → FUNDAMENTAL WAVE → DATA ANALYZING → ANALYSIS RESULT
THE SIGNAL TO BE ANALYZED    EXTRACTING MEANS     MEANS

1

2

FIG. 1

EP 1 039 394 A2

(a) HEARTBEAT SIGNAL CONTAINING NO NOISE

TIME

(b) FUNDAMENTAL WAVE

(c) SIGNAL TO BE ANALYZED

TIME

FIG. 2

EP 1 039 394 A2

(a) SIGNAL TO BE ANALYZED

(b) FUNDAMENTAL WAVE

TIME

FIG. 3

EP 1 039 394 A2

FUNDAMENTAL WAVE h(t)

s(t)

s(t)xh(t)

SAMPLING POINT

SAMPLING POINT

SAMPLING POINT

(a)

(b)

(c)

CORRELATION COEFFICIENT C = 1

FIG. 4

FIG. 5

FIG. 6

CORRELATION COEFFICIENT C = 0.3984

(a)                                    (b)

FIG. 7

EP 1 039 394 A2

FIG. 8

EP 1 039 394 A2

FIG. 9

CORRELATION COEFFICIENT C = 1

(a)

(b)

FIG. 10

NORMALIZED h(t/a)

NORMALIZED s(t)

NORMALIZED s(t) x h(t/a)

(a)

(b)

TIME

TIME

CORRELATION COEFFICIENT C = 0.8957

FIG. 11

EP 1 039 394 A2

(a)

FFT

(b)

FIG. 12

WAVELET FREQUENCY

SAMPLING POINT

(a)

⇩ *FFT*

WAVELET FREQUENCY

FREQUENCY OF THE GREATEST WAVELET COEFFICIENT

(b)

FIG. 13

CONTOUR LINES OBTAINED BY APPLYING FFT TO
THE WAVELET COEFFICIENT OF A 10-15 dB SIGNAL

WAVELET COEFFICIENT FREQUENCY

Pm

(a)

APPLICATION OF FFT TO THE WAVELET
COEFFICIENT OF A 10-15 dB SIGNAL

FREQUENCY

(b)

FIG. 14

SAMPLING POINT

FIG. 15

(a)

SAMPLING POINT

(b)

SAMPLING POINT

(c)

SAMPLING POINT

FIG. 16

(a)

(b)

SAMPLING POINT

SAMPLING POINT

FIG. 17

CONTOUR LINES OF WAVELET COEFFICIENT FOR A SIGNAL
CONTAINING VARIOUS FREQUENCY COMPONENTS

FIG. 18